# EUROPEAN PATENT APPLICATION

(11) **EP 4 368 694 A1**
(43) Date of publication of application: **15.05.2024**
(21) Application number: 22837576.2
(22) Date of filing: 29.06.2022
(51) Int. Cl.: C11D 3/50, C11B 9/00

(54) **PERFUME COMPOSITION**

(30) Priority: 07.07.2021 JP 2021112629
(71) Applicant: Takasago International Corporation, Tokyo 144-8721 (JP)
(72) Inventor: KUWAHARA, Yukari, Hiratsuka-shi, Kanagawa 254-0073 (JP); TAKAHASHI, Kaori, Hiratsuka-shi, Kanagawa 254-0073 (JP); ISHIDA, Kenya, Hiratsuka-shi, Kanagawa 254-0073 (JP)
(74) Representative: Hoffmann Eitle
(86) International application number: PCT/JP2022/026111
(87) International publication number: WO 2023/282160

(57) **Abstract**

The present invention relates to a fragrance composition whose scent, which gives a cooling or warming sensation based on subjective evaluation when the scent is smelt, can actually change a skin temperature, that is, a cooling fragrance that can lower the skin temperature, and a warming fragrance that can increase the skin temperature.

## Description

### TECHNICAL FIELD

The present invention relates to a fragrance composition. Specifically, the present invention relates to a fragrance composition that has sensory stimulation.

### BACKGROUND ART

In the related art, menthol, peppermint oil, vanillyl butyl ether, ginger oil, and the like as fragrance compositions used for the purpose of giving a cooling sensation or a warming sensation have been reported as sensory stimulation fragrance compositions (Patent Literatures 1 to 3 and Non Patent Literature 1).

### CITATION LIST

### PATENT LITERATURE

Patent Literature 1: JP2019-513839A
Patent Literature 2: JP5986713B
Patent Literature 3: JP2011-026613A

### NON-PATENT LITERATURE

Non Patent Literature 1: Aroma Research, (2013), 14(1), P58-63

### SUMMARY OF INVENTION

### TECHNICAL PROBLEM

However, in Patent Literatures 1 to 3 and Non Patent Literature 1, there is no mention of giving a warming sensation or a cooling sensation by sniffing through a nasal cavity and increasing or lowering a skin temperature.

It is known that a fragrance composition used for the purpose of giving a cooling sensation and a warming sensation is generally perceived subjectively through skin contact stimulation, including in the mouth, and does not lower the skin temperature even when coming into contact with the skin. In particular, a receptor that responds to the cooling sensation has been cloned and a mechanism of action has been elucidated. That is, it is known that physical cooling and components such as menthol have the following in common: activating the temperature receptor on the skin surface to excite cold fibers, and creating a sensation that the skin coated with menthol is cold, and do not lower the skin temperature. Similarly, a temperature receptor has been cloned for the warming sensation, and it is known that a similar mechanism causes a sensation that the skin is warm.

### SOLUTION TO PROBLEM

As a result of examining increase or lowering of the skin temperature due to noncontact of a fragrance component to the skin, the present inventors have found a solution consisting of the following composition, which is characterized by giving a warming sensation or a cooling sensation by sniffing through a nasal cavity and increasing or lowering the skin temperature. Thus, the present invention has been completed.

That is, the present invention includes the following contents.
[1] A fragrance composition, which gives a cooling sensation or a warming sensation by sniffing through a nasal cavity and lowers or increases a skin temperature.

### ADVANTAGEOUS EFFECTS OF INVENTION

According to the present invention, it is possible to provide a product that can appeal to a cooling or warming sensation effect not only for personal care and oral care in the related art, but also for a wide range of applications such as air care and fabric care.

### BRIEF DESCRIPTION OF DRAWINGS

[Fig. 1] Fig. 1 is a diagram illustrating a method of measuring a skin temperature.
[Fig. 2] Fig. 2 shows diagrams showing results of a cooling sensation: a change in skin temperature at a nasal tip from a time when a sample is presented.
[Fig. 3] Fig. 3 shows diagrams showing results of a warming sensation: a change in skin temperature at a nasal tip from a time when a sample is presented.

### DESCRIPTION OF EMBODIMENTS

Hereinafter, the present invention will be described in detail.

A fragrance composition according to the present invention can give a warming sensation or a cooling sensation by sniffing through a nasal cavity and can lower or increase a skin temperature.

The fragrance composition according to the present invention that mainly gives a cooling sensation and lowers a skin temperature preferably contains one or more selected from the following fragrance component group A, for example.

### (Fragrance component group A)

2-sec-butylcyclohexanone, L-hydroxycitronellal, menthyl acetate, menthyl lactate, ethyl (1R,6S)-2,2,6-trimethylcyclohexanecarboxylate, borneol, β-caryophyllene, isopulegol, menthone, menthone glycerin acetal, menthyl glyceryl ether, linalool, linalyl acetate, L-menthol, methyl salicylate, 7-methyl-2H-1,5-benzodioxepin-3(4H)-one, 5-cyclohexadecenone, mentha oil, eucalyptus oil, cis-3-hexenol, cis-3-hexenyl acetate, peppermint oil, spearmint oil, and lime oil

In the above component, 2-sec-butylcyclohexanone, L-hydroxycitronellal, and ethyl (1R,6S)-2,2,6-trimethylcyclohexanecarboxylate are preferred. The fragrance of these fragrance components alone and in combination strongly evokes a particularly refreshing and cool feeling, and has a remarkable effect of lowering the skin temperature.

It is preferable that one or more in the fragrance component group A are contained in the fragrance composition according to the present invention. A preferred range of a blending amount of the fragrance component group A in the fragrance composition is preferably 0.1 mass% to 100 mass%, and more preferably 1.0 mass% to 100 mass% in order to exhibit effects of the present invention.

Further, the fragrance component group A can be divided into a fragrance component group A(1) and a fragrance component group A(2), and it is preferable that one or more in each group are contained in the fragrance composition according to the present invention.

### (Fragrance component group A(1))

isopulegol, L-menthol, menthyl acetate, menthyl lactate, menthone, menthone glycerin acetal, menthyl glyceryl ether, and peppermint oil

### (Fragrance component group A(2))

2-sec-butylcyclohexanone, L-hydroxycitronellal, ethyl (1R,6S)-2,2,6-trimethylcyclohexanecarboxylate, 5-cyclohexadecenone, borneol, β-caryophyllene, linalool, linalyl acetate, methyl salicylate, 7-methyl-2H-1,5-benzodioxepin-3(4H)-one, 5-cyclohexadecenone, mentha oil, eucalyptus oil, cis-3-hexenol, cis-3-hexenyl acetate, spearmint oil, and lime oil

A preferred range of a mass ratio (A1)/(A2) of a content (A1) of the fragrance component group A(1) to a content (A2) of the fragrance component group A(2) is 0.01 or more and 0.30 or less in order to exhibit the effects of the present invention.

The fragrance composition according to the present invention that mainly gives a warming sensation and increases a skin temperature preferably contains one or more selected from the following fragrance component group B, for example.

### (Fragrance component group B)

dihydrofarnesal, (R,E)-2-methyl-4-(2,2,3-trimethyl-3-cyclopenten-1-yl)-2-buten-1-ol, (R)-3-methylcyclopentadecanone, vanillyl butyl ether, vanillyl ethyl ether, ethylene brassylate, 2-(2-methyl-1-propenyl)-4-methyltetrahydropyran, vanillin, ethyl vanillin, ethyl maltol, piperonal, γ-nonalactone, cinnamic aldehyde, coumarin, cumin aldehyde, diethyl malonate, ethyl 2-methylvalerate, and ginger oil

Among the above fragrance component group B, the following fragrance components are preferred since the fragrance thereof alone and in combination strongly evokes a particularly comfortable and warming sensation, has a remarkable effect of increasing the skin temperature, and is suitable for use.

dihydrofarnesal, (R,E)-2-methyl-4-(2,2,3-trimethyl-3-cyclopenten-1-yl)-2-buten-1-ol, (R)-3-methylcyclopentadecanone, vanillyl butyl ether, vanillyl ethyl ether, ethylene brassylate, and 2-(2-methyl-1-propenyl)-4-methyltetrahydropyran.

It is preferable that one or more in the fragrance component group B are contained in the fragrance composition according to the present invention. A preferred range of a blending amount of the fragrance component group B in the fragrance composition is preferably 0.1 mass% to 100 mass%, and more preferably 1.0 mass% to 100 mass% in order to exhibit the effects of the present invention.

Further, the fragrance component group B can be divided into a fragrance component group B(1) and a fragrance component group B(2), and it is preferable that one or more in each group are contained in the fragrance composition according to the present invention.

### (Fragrance component group B(1))

vanillyl butyl ether, vanillyl ethyl ether, and ginger oil

### (Fragrance component group B(2))

dihydrofarnesal, (R,E)-2-methyl-4-(2,2,3-trimethyl-3-cyclopenten-1-yl)-2-buten-1-ol, (R)-3-methylcyclopentadecanone, ethylene brassylate, 2-(2-methyl-1-propenyl)-4-methyltetrahydropyran, vanillin, ethyl vanillin, ethyl maltol, piperonal, γ-nonalactone, cinnamic aldehyde, coumarin, cumin aldehyde, diethyl malonate, and ethyl 2-methylvalerate

A preferred range of a mass ratio (B1)/(B2) of a content (B1) of the fragrance component group B(1) to a content (B2) of the fragrance component group B(2) is 0.01 or more and 0.30 or less in order to exhibit the effects of the present invention.

The fragrance composition according to the present invention can contain, as an additional component, at least one or more of a fragrance component that enhances giving of the cooling sensation and lowering of the skin temperature or enhances giving of the warming sensation and increasing of the skin temperature.

Specific examples of the fragrance component that enhances giving of the cooling sensation and lowering of the skin temperature or enhances giving of the warming sensation and increasing of the skin temperature include the following fragrance component group C.

### (Fragrance component group C)

5-mercapto-5-methyl-3-hexanone, 4,7-decadienal, 8-mercaptomenthone, 1-(2,2,6-trimethylcyclohexyl)-3-hexanol, and bergamot oil

Among the above fragrance component group C, 5-mercapto-5-methyl-3-hexanone, 4,7-decadienal, 8-mercaptomenthone, and 1-(2,2,6-trimethylcyclohexyl)-3-hexanol are preferred.

In the above fragrance components, the fragrance thereof alone and in combination strongly evokes a particularly comfortable and cooling sensation or warming sensation, has a remarkable effect of increasing or lowering the skin temperature, and is thus suitable for use.

It is preferable that one or more in the fragrance component group C are contained in the fragrance composition according to the present invention. A preferred range of a blending amount of the fragrance component group C in the fragrance composition is preferably 0.00001 mass% to 30 mass%, and more preferably 0.0001 mass% to 20 mass% in order to exhibit the effects of the present invention.

The fragrance composition according to the present invention can be prepared by appropriately adding and mixing various compounds, and the order and interval of blending are not particularly limited. The formulation form is also not particularly limited and can be appropriately selected depending on the purpose of use.

Known fragrance components or commonly used additives as described below can be incorporated into the fragrance composition according to the present invention within a range that does not impair the effects of the present invention, that is, within a quantitative and qualitative range that can give a cooling sensation and enhances lowering of the skin temperature or give a warming sensation and increases the skin temperature.

Examples of known fragrances include: hydrocarbons such as α-pinene, limonene, and cedrene; aliphatic saturated or unsaturated alcohols such as 1-octen-3-ol and nonanol; saturated or unsaturated chain terpene alcohols such as myrcenol, tetrahydrolinalool, and nerol; cyclic terpene alcohols such as α-terpineol and bornylmethoxycyclohexanol; sesquiterpene alcohols such as farnesol; aromatic alcohols such as γ-phenylpropyl alcohol and dimethylbenzyl carbinol; aliphatic aldehydes; terpene-based aldehydes; aromatic aldehydes; aliphatic ketones such as 7-acetyl-1,2,3,4,5,6,7,8-octahydro-1,1,6,7-tetramethylnaphthalene; terpene cyclic ketones; cyclic ketones; aliphatic esters; acetate esters; propionate esters; butyrate esters; benzoate esters; phenylacetate esters; salicylate esters; and anthranilate esters. Blending amounts of these known fragrance components in the present fragrance composition can be appropriately selected depending on the type of the fragrance to be blended, the purpose, or the like, and are not particularly limited.

The fragrance composition according to the present invention can be blended into, for example, a fragrance product, a detergent, a fabric softener, a cleaner, a kitchen detergent, a bleach, an aerosol agent, a deodorant/fragrant agent, incense, a miscellaneous good, food and beverages, and pharmaceuticals, and gives a cooling sensation and/or a warming sensation through the nasal cavity, and can thus be suitably used in a product not applied directly to the skin or to the mouth.

Examples of the detergent include a heavy laundry detergent, a light laundry detergent, a liquid detergent, a laundry soap, a compact detergent, and a powder soap. Examples of the fabric softener include a softener and a furniture care. Examples of the cleaner include a cleanser, a house cleaner, a toilet cleaner, a bathroom cleaner, a glass cleaner, a mold remover, and a drain cleaner. Examples of the kitchen detergent include a kitchen soap, a synthetic kitchen soap, and a dish detergent. Examples of the bleach include an oxidizing bleach (a chlorine bleach, an oxygen bleach, etc.), a reducing bleach (a sulfur-based bleach, etc.), and an optical bleach. Examples of the aerosol agent include a spray type and a powder spray. Examples of the deodorant/fragrant agent include a solid type, a gel type, and a liquid type. Examples of the miscellaneous good include tissue paper and toilet paper.

When the fragrance composition according to the present invention is blended in the above product, the amount may be determined as appropriate depending on the form and application of the product, and is not particularly limited. The amount is 0.0001 mass% to 100 mass%, preferably 0.001 mass% to 80 mass%, and more preferably 0.01 mass% to 50 mass%, based on a total mass of the product. When the amount is less than 0.0001 mass%, the fragrance component is too few to exhibit the desired cooling and warming sensation performance. When the amount is more than 80 mass%, a sufficient cooling or warming effect can be expected, but the cost may be too high, which may pose a commercial problem.

In addition, an effect of adjusting a sensible temperature and the skin temperature by the fragrance composition according to the present invention and a product containing the same is achieved within 5 minutes immediately after receiving olfactory stimulation, preferably 30 seconds to 4 minutes after receiving olfactory stimulation, and more preferably 1 minutes to 3 minutes immediately after receiving olfactory stimulation. It has been confirmed that when the time is longer than 5 minutes, the warming or cooling effect caused by olfactory stimulation weakens and returns to a steady state in a short period of time, which is a short-term lasting effect.

As detailed above, the present description discloses the following fragrance compositions.
[1] A fragrance composition, which gives a cooling sensation or a warming sensation by sniffing through a nasal cavity and lowers or increases a skin temperature.
[2] The fragrance composition according to [1], containing: at least one or more selected from the following fragrance component group A.
   (Fragrance component group A)
   2-sec-butylcyclohexanone, L-hydroxycitronellal, menthyl acetate, menthyl lactate, ethyl (1R,6S)-2,2,6-trimethylcyclohexanecarboxylate, borneol, β-caryophyllene, isopulegol, menthone, menthone glycerin acetal, menthyl glyceryl ether, linalool, linalyl acetate, L-menthol, methyl salicylate, 7-methyl-2H-1,5-benzodioxepin-3(4H)-one, 5-cyclohexadecenone, mentha oil, eucalyptus oil, cis-3-hexenol, cis-3-hexenyl acetate, peppermint oil, spearmint oil, and lime oil
[3] The fragrance composition according to [1] or [2], in which a mass ratio (A1)/(A2) of a content (A1) of the following fragrance component group A(1) to a content (A2) of the following fragrance component group A(2) is 0.01 or more and 0.3 or less.
   (Fragrance component group A(1))
      isopulegol, L-menthol, menthyl acetate, menthyl lactate, menthone, menthone glycerin acetal, menthyl glyceryl ether, and peppermint oil
   (Fragrance component group A(2))
      2-sec-butylcyclohexanone, L-hydroxycitronellal, ethyl (1R,6S)-2,2,6-trimethylcyclohexanecarboxylate, 5-cyclohexadecenone, borneol, β-caryophyllene, linalool, linalyl acetate, methyl salicylate, 7-methyl-2H-1,5-benzodioxepin-3(4H)-one, mentha oil, eucalyptus oil, cis-3-hexenol, cis-3-hexenyl acetate, spearmint oil, and lime oil
[4] The fragrance composition according to [1], containing: at least one or more selected from the following fragrance component group B.
   (Fragrance component group B)
   dihydrofarnesal, (R,E)-2-methyl-4-(2,2,3-trimethyl-3-cyclopenten-1-yl)-2-buten-1-ol, (R)-3-methylcyclopentadecanone, vanillyl butyl ether, vanillyl ethyl ether, ethylene brassylate, 2-(2-methyl-1-propenyl)-4-methyltetrahydropyran, vanillin, ethyl vanillin, ethyl maltol, piperonal, γ-nonalactone, cinnamic aldehyde, coumarin, cumin aldehyde, diethyl malonate, ethyl 2-methylvalerate, and ginger oil
[5] The fragrance composition according to [1] or [4], in which a mass ratio (B1)/(B2) of a content (B1) of the following fragrance component group B(1) to a content (B2) of a fragrance component group B(2) is 0.01 or more and 0.30 or less.
   (Fragrance component group B(1))
      vanillyl butyl ether, vanillyl ethyl ether, and ginger oil
   (Fragrance component group B(2))
      dihydrofarnesal, (R,E)-2-methyl-4-(2,2,3-trimethyl-3-cyclopenten-1-yl)-2-buten-1-ol, (R)-3-methylcyclopentadecanone, ethylene brassylate, 2-(2-methyl-1-propenyl)-4-methyltetrahydropyran, vanillin, ethyl vanillin, ethyl maltol, piperonal, γ-nonalactone, cinnamic aldehyde, coumarin, cumin aldehyde, diethyl malonate, and ethyl 2-methylvalerate
[6] The fragrance composition according to any one of [1] to [5], further containing: one or more selected from the following fragrance component group C.
   (Fragrance component group C)
   5-mercapto-5-methyl-3-hexanone, 4,7-decadienal, 8-mercaptomenthone, 1-(2,2,6-trimethylcyclohexyl)-3-hexanol, and bergamot oil
[7] The fragrance composition according to any one of [1] to [6], further containing: one or more selected from the following fragrance component group C, to enhance giving of the cooling sensation and lowering of the skin temperature or giving of the warming sensation and increasing of the skin temperature.
   (Fragrance component group C)
   5-mercapto-5-methyl-3-hexanone, 4,7-decadienal, 8-mercaptomenthone, 1-(2,2,6-trimethylcyclohexyl)-3-hexanol, and bergamot oil
[8] The fragrance composition according to any one of [1] to [7], in which a sensible temperature and the skin temperature are adjusted by olfactory stimulation due to the fragrance composition, and the adjustment effect is developed and maintained within 5 minutes immediately after receiving the olfactory stimulation.
[9] A product containing: the fragrance composition according to any one of [1] to [8] in an amount of 0.0001 mass% to 100 mass%.
[10] A product containing: the fragrance composition according to any one of [1] to [8] in an amount of 0.0001 mass% to 100 mass%, in which a product applied directly to skin and a product applied directly to mouth are excluded.
[11] A method of adjusting a sensible temperature and a skin temperature by olfactory stimulation using the fragrance composition according to any one of [1] to [8] or the product according to [9] or [10].

### Examples

Hereinafter, the present invention will be specifically described with reference to Examples, but the present invention is not limited to these in any way, and various changes and modifications may be made without departing from the scope of the present invention.

### [Example 1]

A cooling fragrance composition was prepared according to a blending formulation shown in Table 1. The unit of the amount of the blended component is mass%.

### [Table 1]

**Table 1**

| | |
|---|---|
| 5-Cyclohexadecenone | 10 |
| 2-Sec-butylcyclohexanone | 6 |
| L-hydroxycitronellal | 10 |
| Menthone | 6 |
| Menthyl acetate | 0.45 |
| Ethyl (1R,6S)-2,2,6-trimethylcyclohexanecarboxylate | 0.3 |
| Dipropylene glycol | 67.25 |
| Total | 100 |

### [Example 2]

A cooling fragrance composition was prepared according to a blending formulation shown in Table 2. The unit of the amount of the blended component is mass%.

### [Table 2]

**Table 2**

| | |
|---|---|
| Borneol | 8 |
| β-Caryophyllene | 0.5 |
| Isopulegol | 1 |
| Linalool | 10 |
| Linalyl acetate | 10 |
| L-menthol | 15 |
| Methyl salicylate | 0.2 |
| 7-Methyl-2H-1,5-benzodioxepin-3(4H)-one | 0.1 |
| Mentha oil | 8.5 |
| Eucalyptus oil | 1 |
| Cis-3-hexenol | 5 |
| Cis-3-hexenyl acetate | 5 |
| Lime oil | 0.5 |
| Peppermint oil | 0.5 |
| Spearmint oil | 0.5 |
| Dipropylene glycol | 34.2 |
| Total | 100 |

### [Comparative Example 1]

A fragrance composition was prepared according to a blending formulation shown in Table 3. The unit of the amount of the blended component is mass%.

### [Table 3]

**Table 3**

| | |
|---|---|
| Benzyl salicylate | 20 |
| Benzyl alcohol | 10 |
| Ethyl salicylate | 5 |
| Hexyl 2-methylbutyrate | 5 |
| β-Pinene | 5 |
| Dipropylene glycol | 55 |
| Total | 100 |

### [Comparative Example 2]

A fragrance composition was prepared according to a blending formulation shown in Table 4. The unit of the amount of the blended component is mass%.

### [Table 4]

**Table 4**

| | |
|---|---|
| Camphor | 10 |
| Hexyl salicylate | 10 |
| Orange oil | 10 |
| Allyl heptoate | 10 |
| Styrallyl acetate | 10 |
| Dipropylene glycol | 50 |
| Total | 100 |

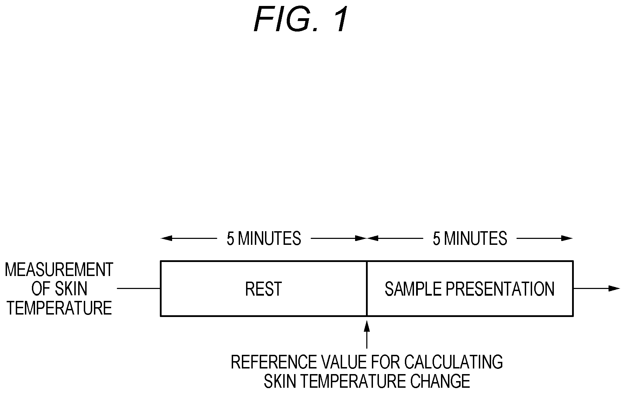

The effects of the fragrance compositions in Examples 1 and 2 and Comparative Examples 1 and 2 were determined by seven panelists using a peripheral skin temperature measurement method and subjective evaluation described below. Fig. 1 shows a method of measuring a skin temperature as a simplified diagram.

The peripheral skin temperature upon presentation of the fragrance composition was measured using a thermistor-type high-performance thermometer (LT-200SA, gram Inc.). A thermistor sensor was fixed to a nasal tip to measure a change in skin temperature at the nasal tip. The measurement was performed after 20 minutes or longer of acclimatization in a laboratory at 25°C. After a resting time as a control, a change in skin temperature was measured when a sample was presented. Experiment participants first rested for 5 minutes and then were exposed to 3.0 g of the sample presented to the tip of their nose for 5 minutes. The sample was presented in a light-shielding glass bottle at a position approximately 10 cm in front of the nasal cavity of the experimental participant.

A change in peripheral skin temperature was calculated by comparing the skin temperature at the start of sample presentation as a reference value with the skin temperature 150 seconds after sample presentation.

The subjective evaluation was performed after the sample presentation. The average value of sensory evaluation was calculated using the following evaluation criteria.

### Cooling or warming sensation of scent

5: a very strong cooling sensation or warming sensation is felt.
4: a strong cooling sensation or warming sensation is felt.
3 : a cooling sensation or warming sensation is felt.
2: a slight cooling sensation or warming sensation is felt.
1: a little cooling sensation or warming sensation is felt.
0: no cooling sensation or warming sensation is felt.

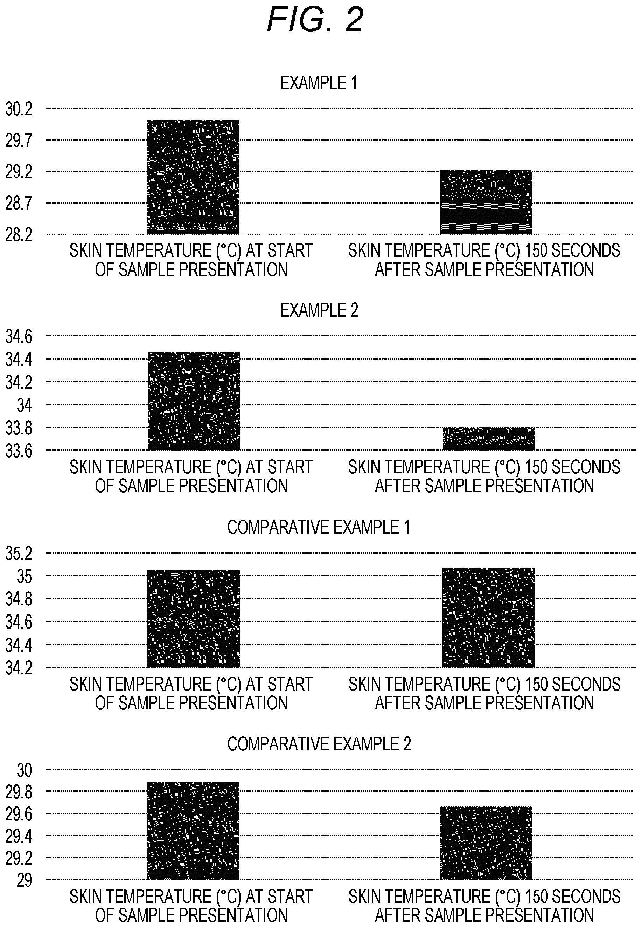

The test results are shown in Fig. 2 and Table 5. Accordingly, it is confirmed that Examples 1 and 2 containing a cooling sensation composition lower the peripheral skin temperature and give a cooling sensation from the scent.

### [Table 5]

**Table 5**

| | Example 1 | Example 2 | Comparative Example 1 | Comparative Example 2 |
|---|---|---|---|---|
| Skin temperature (°C) at start of sample presentation | 30.02 | 34.46 | 35.05 | 29.88 |
| Skin temperature (°C) 150 seconds after sample presentation | 29.21 | 33.79 | 35.06 | 29.66 |
| Difference in skin temperature (°C) | -0.81 | -0.67 | 0.01 | -0.22 |
| Subjective evaluation of scent | 3.8 | 2.6 | 0.5 | 2.5 |

### [Example 3]

A test same as in Example 1 was performed by adding 0.001% of 8-mercaptomenthone and 0.1% of 1-(2,2,6-trimethylcyclohexyl)-3-hexanol from the fragrance component group C to the fragrance composition in Example 1. As a result, the peripheral skin temperature further decreased by 0.2°C and 1.01°C, and the subjective evaluation score improved by 0.2 to 4.0.

### [Example 4]

A warming fragrance composition was prepared according to a blending formulation shown in Table 6. The unit of the amount of the blended component is mass%.

### [Table 6]

**Table 6**

| | |
|---|---|
| Dihydrofarnesal | 17 |
| (R)-2-methyl-4-(2,2,3-trimethyl-3-cyclopenten-1-yl)-2-buten-1-ol | 15 |
| (R)-3-methylcyclopentadecanone | 0.5 |
| Vanillyl butyl ether | 10 |
| Vanillyl ethyl ether | 10 |
| Ethylene brassylate | 10 |
| Dipropylene glycol | 37.5 |
| Total | 100 |

### [Example 5]

A warming fragrance composition was prepared according to a blending formulation shown in Table 7. The unit of the amount of the blended component is mass%.

### [Table 7]

**Table 7**

| | |
|---|---|
| 2-(2-Methyl-1-propenyl)-4-methyltetrahydropyran | 1 |
| Vanillin | 0.5 |
| Ethyl maltol | 5 |
| Ethyl vanillin | 0.5 |
| Piperonal | 7 |
| γ-Nonalactone | 8 |
| Cinnamic aldehyde | 0.5 |
| Coumarin | 12 |
| Cumin aldehyde | 1 |
| Diethyl malonate | 20 |
| Ethyl 2-methylvalerate | 20 |
| Ginger oil | 0.5 |
| Dipropylene glycol | 24 |
| Total | 100 |

### [Comparative Example 3]

A fragrance composition was prepared according to a blending formulation shown in Table 8. The unit of the amount of the blended component is mass%.

### [Table 8]

**Table 8**

| | |
|---|---|
| 1-(2,2,6-Trimethylcyclohexa-1,3-dienyl)-2-buten-1-one | 1 |
| Geraniol | 5 |
| Methyl cinnamic aldehyde | 6 |
| 1-(2,6,6-Trimethyl-3-cyclohexen-1-yl)-2-buten-1-one | 10 |
| Orange oil | 10 |
| Dihydroeugenol | 15 |
| 1,3,4,6,7,8-Hexahydro-4,6,6,7,8,8-hexamethylcyclopenta-γ-2-benzopyran | 20 |
| Methyl dihydrojasmonate | 20 |
| Dipropylene glycol | 13 |
| Total | 100 |

### [Comparative Example 4]

A fragrance composition was prepared according to a blending formulation shown in Table 9. The unit of the amount of the blended component is mass%.

### [Table 9]

**Table 9**

| | |
|---|---|
| γ-Methylionone | 20 |
| Phenylethyl alcohol | 20 |
| γ-Undecalactone | 10 |
| 2-Tert-butylcyclohexyl acetate | 20 |
| Dipropylene glycol | 30 |
| Total | 100 |

The effects of Examples 4 and 5 and Comparative Examples 3 and 4 were determined by seven panelists using a peripheral skin temperature measurement method and subjective evaluation similar to Example 1. The peripheral skin temperature and the subjective evaluation were measured in the same manner as described above.

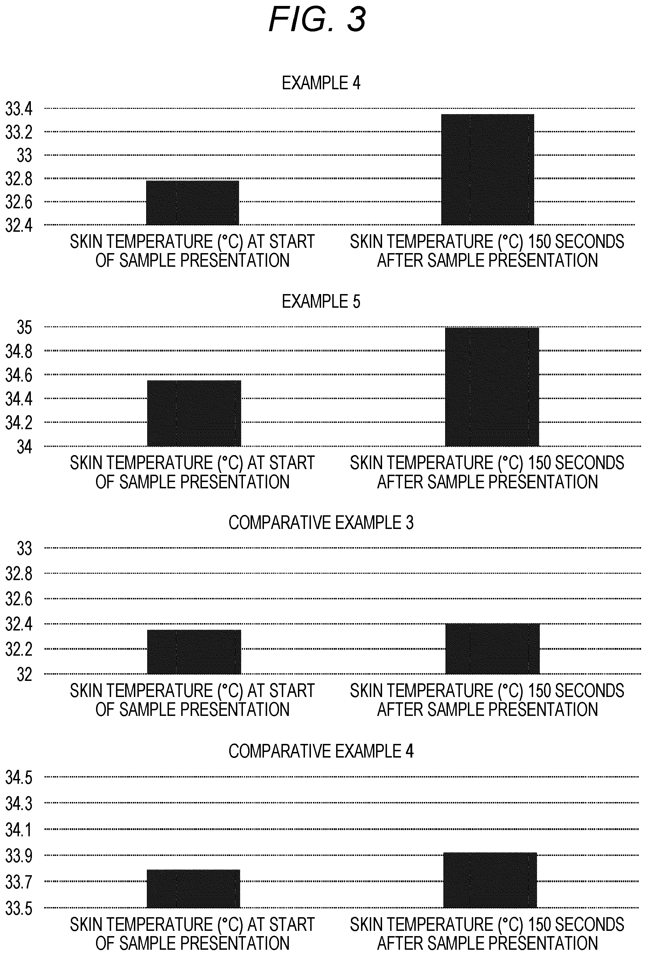

The test results are shown in Fig. 3 and Table 10. Accordingly, it is confirmed that Examples 4 and 5 containing a warming sensation composition increase the peripheral skin temperature and give a warming sensation from the scent.

### [Table 10]

**Table 10**

| | Example 4 | Example 5 | Comparative Example 3 | Comparative Example 4 |
|---|---|---|---|---|
| Skin temperature (°C) at start of sample presentation | 32.78 | 34.55 | 32.35 | 33.79 |
| Skin temperature (°C) 150 seconds after sample presentation | 33.35 | 34.99 | 32.40 | 33.92 |
| Difference in skin temperature (°C) | +0.57 | +0.44 | 0.05 | 0.13 |
| Subjective evaluation of scent | 4.0 | 3.2 | 0.3 | 2.4 |

### [Example 6]

Further, 0.01% of 5-mercapto-5-methyl-3-hexanone and 5% of lime oil were added to the fragrance composition in Example 4 from the fragrance compound group C to present a scent. As a result, the peripheral skin temperature further increased by 0.1°C, and the subjective evaluation score improved by 0.2.

### [Example 7] Cooling fragrance composition

A cooling fragrance composition was prepared according to a blending formulation shown in Table 11. The unit of the amount of the blended component is mass%.

### [Table 11]

**Table 11**

| | |
|---|---|
| 5-Cyclohexadecenone | 10 |
| 5-Mercapto-5-methyl-3-hexanone (100 ppm/dipropylene glycol solution) | 0.5 |
| Linalyl acetate | 10 |
| Menthol | 1.5 |
| Menthyl acetate | 0.45 |
| Isopulegol | 1.5 |
| 2-Sec-butylcyclohexanone | 10 |
| Ethyl (1R,6S)-2,2,6-trimethylcyclohexanecarboxylate | 0.3 |
| Bergamot oil | 3 |
| Peppermint oil | 3 |
| Dipropylene glycol | 59.75 |
| Total | 100 |

### [Example 8] Liquid detergent

A liquid detergent was prepared according to a formulation shown in Table 12 below.

### [Table 12]

**Table 12**

| Component | Blending amount (g) |
|---|---|
| Polyoxyalkylene alkyl ether | 50 |
| Water | 35.13 |
| Butyl carbitol | 8 |
| Linear alkylbenzene sulfonate | 2.5 |
| p-Toluenesulfonic acid | 1 |
| Polyethylene glycol | 1 |
| Monoethanolamine | 1 |
| Coconut oil fatty acid | 0.1 |
| Citric acid | 0.22 |
| Sodium benzoate | 0.5 |
| BHT | 0.05 |
| Cooling fragrance composition in Example 7 (Table 11) | 0.5 |
| Total | 100 |

### [Example 9] Clothing softener

A clothing softener was prepared according to a formulation shown in Table 13 below.

### [Table 13]

**Table 13**

| Component | Blending amount (g) |
|---|---|
| Dialkyldimethylammonium chloride | 15 |
| POE (30) lauryl ether | 3 |
| Fatty acid | 1 |
| Dimethylpolysiloxane | 0.5 |
| Ethylene glycol | 5 |
| Preservative | Appropriate amount |
| Sequestering agent | Appropriate amount |
| Cooling fragrance composition in Example 7 (Table 11) | 1 |
| Ion exchange water | Balance |
| Total | 100 |

### [Example 10] Warming fragrance composition

A warming fragrance composition was prepared according to a blending formulation shown in Table 14. The unit of the amount of the blended component is mass%.

### [Table 14]

**Table 14**

| | |
|---|---|
| Dihydrofarnesal | 17 |
| 1-(2,2,6-Trimethylcyclohexyl)-3-hexanol | 0.1 |
| (R,E)-2-methyl-4-(2,2,3-trimethyl-3-cyclopenten-1-yl)-2-buten-1-ol | 15 |
| (R)-3-methylcyclopentadecanone | 0.5 |
| Bergamot oil | 10 |
| Vanillyl ethyl ether | 10 |
| Ethylene brassylate | 10 |
| Dipropylene glycol | 37.4 |
| Total | 100 |

### [Example 11] Fragrant agent

A fragrant agent was prepared according to a formulation shown in Table 15 below.

### [Table 15]

**Table 15**

| Component | Blending amount (g) |
|---|---|
| Ethanol | 10 |
| Isoparaffin | 75 |
| Warming fragrance composition in Example 10 (Table 14) | 15 |
| Total | 100 |

Although the present invention has been described in detail and with reference to specific embodiments, it will be apparent to those skilled in the art that various changes and modifications can be made without departing from the spirit and scope of the present invention. This application is based on a Japanese Patent Application (No. 2021-112629) filed on July 7, 2021, the contents of which are incorporated herein as a reference.

## Claims

1. A fragrance composition, which gives a cooling sensation or a warming sensation by sniffing through a nasal cavity and lowers or increases a skin temperature.

2. The fragrance composition according to claim 1, comprising: at least one or more selected from the following fragrance component group A.
(Fragrance component group A)
2-sec-butylcyclohexanone, L-hydroxycitronellal, menthyl acetate, menthyl lactate, ethyl (1R,6S)-2,2,6-trimethylcyclohexanecarboxylate, borneol, β-caryophyllene, isopulegol, menthone, menthone glycerin acetal, menthyl glyceryl ether, linalool, linalyl acetate, L-menthol, methyl salicylate, 7-methyl-2H-1,5-benzodioxepin-3(4H)-one, 5-cyclohexadecenone, mentha oil, eucalyptus oil, cis-3-hexenol, cis-3-hexenyl acetate, peppermint oil, spearmint oil, and lime oil

3. The fragrance composition according to claim 1, wherein a mass ratio (A1)/(A2) of a content (A1) of the following fragrance component group A(1) to a content (A2) of the following fragrance component group A(2) is 0.01 or more and 0.3 or less.
(Fragrance component group A(1))
isopulegol, L-menthol, menthyl acetate, menthyl lactate, menthone, menthone glycerin acetal, menthyl glyceryl ether, and peppermint oil
(Fragrance component group A(2))
2-sec-butylcyclohexanone, L-hydroxycitronellal, ethyl (1R,6S)-2,2,6-trimethylcyclohexanecarboxylate, 5-cyclohexadecenone, borneol, β-caryophyllene, linalool, linalyl acetate, methyl salicylate, 7-methyl-2H-1,5-benzodioxepin-3(4H)-one, mentha oil, eucalyptus oil, cis-3-hexenol, cis-3-hexenyl acetate, spearmint oil, and lime oil

4. The fragrance composition according to claim 1, comprising: at least one or more selected from the following fragrance component group B.
(Fragrance component group B)
dihydrofarnesal, (R,E)-2-methyl-4-(2,2,3-trimethyl-3-cyclopenten-1-yl)-2-buten-1-ol, (R)-3-methylcyclopentadecanone, vanillyl butyl ether, vanillyl ethyl ether, ethylene brassylate, 2-(2-methyl-1-propenyl)-4-methyltetrahydropyran, vanillin, ethyl vanillin, ethyl maltol, piperonal, γ-nonalactone, cinnamic aldehyde, coumarin, cumin aldehyde, diethyl malonate, ethyl 2-methylvalerate, and ginger oil

5. The fragrance composition according to claim 1, wherein a mass ratio (B 1)/(B2) of a content (B 1) of the following fragrance component group B(1) to a content (B2) of a fragrance component group B(2) is 0.01 or more and 0.30 or less.
(Fragrance component group B(1))
vanillyl butyl ether, vanillyl ethyl ether, and ginger oil
(Fragrance component group B(2))
dihydrofarnesal, (R,E)-2-methyl-4-(2,2,3-trimethyl-3-cyclopenten-1-yl)-2-buten-1-ol, (R)-3-methylcyclopentadecanone, ethylene brassylate, 2-(2-methyl-1-propenyl)-4-methyltetrahydropyran, vanillin, ethyl vanillin, ethyl maltol, piperonal, γ-nonalactone, cinnamic aldehyde, coumarin, cumin aldehyde, diethyl malonate, and ethyl 2-methylvalerate

6. The fragrance composition according to claim 1, comprising: one or more selected from the following fragrance component group C.
(Fragrance component group C)
5-mercapto-5-methyl-3-hexanone, 4,7-decadienal, 8-mercaptomenthone, 1-(2,2,6-trimethylcyclohexyl)-3-hexanol, and bergamot oil

7. The fragrance composition according to claim 1, comprising: one or more selected from the following fragrance component group C, to enhance giving of the cooling sensation and lowering of the skin temperature or giving of the warming sensation and increasing of the skin temperature.
(Fragrance component group C)
5-mercapto-5-methyl-3-hexanone, 4,7-decadienal, 8-mercaptomenthone, 1-(2,2,6-trimethylcyclohexyl)-3-hexanol, and bergamot oil

8. The fragrance composition according to claim 1, wherein a sensible temperature and the skin temperature are adjusted by olfactory stimulation due to the fragrance composition, and the adjustment effect is developed and maintained within 5 minutes immediately after receiving the olfactory stimulation.

9. A product comprising: the fragrance composition according to any one of claims 1 to 8 in an amount of 0.0001 mass% to 100 mass%.

10. A product comprising: the fragrance composition according to any one of claims 1 to 8 in an amount of 0.0001 mass% to 100 mass%, wherein a product applied directly to skin and a product applied directly to mouth are excluded.

11. A method of adjusting a sensible temperature and a skin temperature by olfactory stimulation using the fragrance composition according to any one of claims 1 to 8 or the product according to claim 9.
